# EUROPÄISCHE PATENTSCHRIFT

(11) **EP 2 801 346 B1**
(45) Veröffentlichungstag und Bekanntmachung des Hinweises auf die Patenterteilung: **25.05.2016**
(21) Anmeldenummer: 14000719.6
(22) Anmeldetag: 28.02.2014
(51) Int. Cl.: A61K 8/24, A61K 8/41, A61C 17/00, A61Q 11/00, A61K 8/96, A61K 8/21, A61K 8/37, A61K 8/34, A61K 8/44, A61K 8/73

(54) **Besonders lagerstabile und thixotrop standfeste Prophylaxepaste für den professionellen zahnärztlichen Gebrauch**
Particularly storage stable and thixotropic prophylaxis paste for professional dental use
Pâte prophylactique particulièrement stable au stockage et thixotropiquement résistante pour une utilisation en dentisterie professionnelle

(30) Priorität: 11.03.2013 DE 102013004088
(43) Veröffentlichungstag der Anmeldung: 12.11.2014
(73) Patentinhaber: VOCO GmbH, 27472 Cuxhaven (DE)
(72) Erfinder: Lübbe, Gerrit, 27476 Cuxhaven (DE); Plaumann, Manfred Thomas, 27474 Cuxhaven (DE)

(56) Entgegenhaltungen:
- EP-A1- 2 286 786
- US-A1- 2011 059 420

## Beschreibung

Die Erfindung betrifft Zahnpflegemittel zur vorbeugenden Zahnhygiene, die von Zahnärzten in der Regel in einem mehrmonatigen, halbjährigen oder jährigen Reinigungszyklus am Patienten angewendet werden kann. Die Zusammensetzungen dieser Zahnpflegemittel, die auch Prophylaxepasten genannt werden, unterscheiden sich von denen herkömmlicher Zahnpasten, die ohne professionelle Unterstützung von allen Menschen im Allgemeinen zweimal täglich in der Mundhöhle verwendet werden.
Die Erfindung betrifft ferner Kits, umfassend die erfindungsgemäßen Prophylaxepasten, Verfahren zur Herstellung der Prophylaxepasten sowie die Verwendung der erfindungsgemäßen Zusammensetzungen zur Herstellung der Prophylaxepasten.

Während Zahnpasten der Entfernung von Nahrungsmittelresten dienen und dem Verbraucher ein erfrischendes Mundgefühl geben, dienen die Prophylaxepasten dem Polieren von Zähnen, der Verhinderung von Plaquebildung, Karies und Gingivitis oder der Zahnsteinbehandlung, der Entfernung von Zahnfleckenverfärbungen und von hartnäckig an den Zähnen anhaftenden Belägen sowie der Verhinderung der Entwicklung empfindlicher Zähne. Weitere Indikationen betreffen die Reinigung und Politur von Füllungen, das Polieren der Zahnoberflächen vor dem Bleichen oder nach der Entfernung von KFO-Geräten im Rahmen einer professionellen Zahnreinigung sowie das Entfernen von Resten von temporärem Befestigungsmaterial vor dem definitiven Einkleben.

Pellikel, die sich als dünne Filme nach dem Zähneputzen auf die Zahnoberflächen legen und aus Speichelproteinen gebildet werden, ermöglichen das Anhaften bestimmter Bakterien und die Ausbildung von Biofilmen. Zahnbelag entsteht auf diesen Filmen. Eine fachmännische Politur mit einer entsprechenden Prophylaxepaste unterbindet das Biofilmwachstum, zerstört den Film und stellt sicher, dass auf der polierten, glatten Zahnoberfläche eine schnelle, erneute Biofilmbildung vorerst unterbleibt. Allerdings muß sichergestellt werden, dass auf der einen Seite die Reinigungs- und Polierwirkung der Paste optimal entwickelt ist und auf der anderen Seite ein gesundheitsschädlicher Abtrag und eine Schädigung der Zahnoberflächen minimiert werden.

Prophylaxepasten, die durch den Zahnarzt appliziert werden, stellen somit eine wichtige Ergänzung zum täglichen Zähneputzen dar. Die zusätzliche zahnärztliche Behandlung soll die Zahnpflege so unterstützen, dass Karies verringert und weitere therapeutische Maßnahmen überflüssig werden.

Prophylaxepasten bestehen in der Regel aus drei unterschiedlichen, nach Härtegraden unter-teilten Pasten. In der zahnärztlichen Praxis kann bei Durchführung des Prophylaxeverfahrens die Zahnoberfläche zunächst mit der harten, dann mit der mittelharten und schließlich mit der weichen Paste behandelt werden. Im ersten Arbeitsgang erfolgt ein Aufrauhen der Zahnoberfläche mit der harten Paste. Die Fluoridkomponente der Paste wird in die rauhen Zahnoberflächen eingelegt. Die mittelharte und weiche Paste poliert dann die groben Unebenheiten auf den Zähnen plan.

Alternativ können die verschieden harten Pasten, die unterschiedlichen Reinigungsstärken entsprechen, auch einzeln am Patienten angewendet werden. Hierbei kann dann individuell, je nach Grad der Zahnverunreinigungen, auf die einzelne Patientensituation eingegangen werden. Aber auch in diesen Fällen sollte nach Anwendung einer Paste mit einer höheren Reinigungsstärke eine abschließende Behandlung mit einer weichen Paste erfolgen.

Prophylaxepasten sind aus dem Stand der Technik bekannt.

US 2011/0059420 A1 beschreibt eine dentale Prophylaxepaste, die ausgezeichnete Fließeigenschaften während der Herstellung, eine gute Anwendbarkeit am Patienten und Remineralisierungseigenschaften aufweist. Die Paste enthält wenigstens ein wasserlösliches Calziumsalz, ein wasserlösliches Phosphatsalz, eine wasserlösliche Fluoridverbindung, eine Silikatmatrix, Wasser und ein Feuchtigkeitshaltemittel.

In WO 2009/140577 A1 werden orale Zusammensetzungen angegeben, wobei amorphe Kieselsäuren mit durchschnittlichen Teilchengrößen von 5 µm bis 20 µm und einer Einlehner Härte von 4 bis 11 als Putzmittel verwendet werden.

Aus der EP 1 278 506 B1 sind Zusammensetzungen von Prophylaxepasten bekannt, die unter Verwendung eines Abrasivstoffes aus Borsilikatglas bestimmter Zusammensetzung mit Teilchengrößen kleiner 200 µm und einem mittleren Durchmesser kleiner 50 µm herstellbar sind.

In der US 6,280,707 B1 werden dentale Prophylaxepasten mit antimikrobiellen Eigenschaften offenbart. Die Pasten enthalten als antimikrobiellen Wirkstoff Triclosan.

US 2002/0098156 A1 beschreibt Zusammensetzungen von Prophylaxepasten, in denen als Abrasivmittel Roxite Teilchen mit durchschnittlichen Größen von kleiner 5 µm verwendet werden. Roxite ist ein auf Zirkoniumoxid basierendes Polierpulver, das bevorzugt in Größen kleiner 1,3 µm eingesetzt wird.

EP 1 938 786 B1 offenbart Prophylaxepasten, die als alleinigen Reinigungs- und Polierkörper expandiertes Perlit mit einer mittleren Korngröße von etwa 20 µm bis etwa 70 µm und Schwebeteilchen aufweisen. Die Zusammensetzungen enthalten das Perlit mit einem Darcy-Wert im Bereich von etwa 0,1 bis etwa 3 in Mengen von 40 bis 55 Gew.-%, etwa 5 Gew.-% oder weniger von einem Tensid und mehr als 10 Gew.-% Wasser.

DE 692 04 942 T2 beansprucht eine Prophylaxepaste, die als alleinigen, kombinierten Reinigungs- und Polierkörper ein feinteiliges Gestein mit scharfkantigen Teilchen enthält, das unter Anwendungsbedingungen in kleinere, ebenfalls scharfkantige Teilchen zerfällt und weiterhin 1,2 Propandiol sowie ein Benetzungsmittel enthält, wobei die Paste praktisch wasserfrei ist. Als feinteiliges Gestein wird Perlit verwendet.

In US 7,332,150 B2 werden Prophylaxepasten geschützt, die Putzkörper aus Borsilikatglas mit planaren Körpern in Größen von 45 bis 300 µm und einer Dicke von ca. 4 µm aufweisen, wobei sich die Glasplättchen bei Ausübung von Druck parallel zur oralen Oberfläche ausrichten und so ihre abrasive Wirkung reduziert wird.

US 6,280,707 B1 beschreibt Prophylaxepasten bestehend aus Triclosan als antibakteriellem Zusatz, Wasser, einem Feuchthaltemittel und einem Polierkörper, der ausgewählt ist aus der Gruppe bestehend aus Bimsstein und Ton sowie Mischungen von Bimsstein und Ton.

DE 602 10 674 T2 gibt ein Oberflächenbehandlungsverfahren zum Abtrag einer Zahnoberflächenbeschichtung an, im dem ein Löschagens angewendet wird, das eine Vielzahl von Partikeln umfasst, wobei die Partikel ein Präzipitat oder Agglomerat von Kalziumcarbonat beinhalten, wobei das Verfahren den Schritt des Kontaktierens der Oberfläche mit dem Löschagens beinhaltet, so dass zumindest einige der Partikel zumindest einen Abschnitt der Oberfläche entlangrollen, wobei ein Einfallswinkel der Partikel und der Oberfläche zwischen 0° und 60° liegt, wobei die Partikel im Allgemeinen rund sind und eine unregelmäßige Oberflächenkonfiguration aufweisen, um eine zu rollende Bewegung entlang der Oberfläche herbeizuführen, so dass die Partikel an der Beschichtung reiben und sie absorbieren und wobei das Löschagens im Wesentlichen nicht wässrig ist und wobei die Partikel einen durchschnittlichen maximalen Durchmesser von zwischen 30 und 1000 m aufweisen. Die Verwendung dieses Verfahrens ist u.a. zur Entfernung von Zahnbelag und zur allgemeinen Reinigung und zum Polieren von Zähnen und Prothesen vorgesehen.

US 2011/0008270 betrifft eine wasserlösliche Zusammensetzung als Prophylaxepaste, enthaltend eine wassermischbare Flüssigkeit und wenigstens 10 Gew.-% wasserlösliche Teilchen, die ausgewählt sind aus der Gruppe bestehend aus organischen Säuren und deren Salze, Aminosäuren und deren Salze, Monosacchariden, Disacchariden und Mischungen dieser Komponenten, wobei die Zusammensetzung im Wesentlichen frei von Wasser ist und die Gew.-% sich auf die gesamte Zusammensetzung beziehen.

WO 2005/027863 zielt auf ein Mund- und Zahnpflegemittel als Polier- oder Putzmittel, enthaltend ein Kompositmaterial, enthaltend in Wasser schwerlösliche Calziumsalze in Form von nanopartikulären Primärteilchen mit einer Länge von 5 bis 150 nm und einem Querschnitt von 2 bis 50 nm und Proteinkomponenten, ausgewählt aus Proteinen, Proteinhydrolysaten und Proteinhydrolysat-Derivaten und 0,1 bis 9 Gew.-% eines Putzmittels, bezogen auf das Gesamtgewicht des Mittels.

WO 2010/009133 A2 beschreibt eine dentale Prophylaxezusammensetzung, bei deren Anwendung die Zahnoberfläche keinen mechanischen Abrieb aufweisen soll und wobei das nichtabrasive Poliermittel Chlordioxid umfasst.

US 2010/0135925 A1 gibt Prophylaxepasten mit Putzkörpern auf der Basis von Quarzglas an. Das Quarzglas ist eine amorphe Kieselsäure hoher Reinheit und weist eine mittlere Teilchengröße von wenigstens 7 µm auf. Die Zusammensetzungen sollen PCR (pellicle cleaning ratio) Werte von wenigstens 100 erbringen.

In DE 600 35 344 T2 wird eine Anti-Sensibilitätsdentalmasse offenbart, die auch in einer Prophylaxepaste zum Polieren von Zähnen oder zur Behandlung empfindlicher Zähne oder zur Verhinderung der Entwicklung empfindlicher Zähne nach einer Zahnsteinbehandlung, Wurzelreinigung bzw. Glättung oder Fleckenentfernung durch den Zahnarzt oder Hygieniker verwendet werden kann.

EP 0 268 763 beschreibt eine Prophylaxepaste, die als Putzkörper eine Mischung, bestehend aus einem Perlit und einer synthetisch hergestellten Fällungskieselsäure enthält, wobei man die Fällungskieselsäure dadurch erhält, dass man die in üblicher Weise auf dem Fällungswege erhaltenen Fällungskieselsäuren unterschiedlicher Teilchengröße und Teilchendichte in der Suspensionsphase homogen miteinander vermischt und die Gemische in üblicher Weise durch Filtrieren, Waschen, Trocknen und Vermahlen aufarbeitet.

EP 1 051 962 B1 beansprucht eine Prophylaxepaste, die plättchenförmige, ebene oder gekrümmte Partikel eines Gesteins, insbesondere von Perlit, als hauptsächlichen Bestandteil des Putzkörpers und einen Anteil von 20 - 80 Gew.-% an Polyether aufweist, Putzkörper und Polyether zusammen mindestens 30 Gew.-% des Zahnpflegemittels darstellen und 0 - 20 Gew.-% Emulgator vorhanden sind, wobei wenigstens der Polyetheranteil zur Einstellung der Fließeigenschaften dient.

In der EP 2 286 786 A1 wird ein Zahnpflegemittel zur Remineralisierung von Zähnen vorgeschlagen, aufweisend einen Wirkstoffkomplex aus Hydroxylapatit und Fluorid, wobei der Wirkstoffkomplex außerdem Xylitol zur angeblichen Erhöhung der Remineralisierungswirkung durch das Fluorid aufweist.

Der Hauptteil des Standes der Technik bei dentalen Prophylaxepasten ist auf das wichtige Problem gerichtet, einen Putzkörper bereitzustellen, der die Zähne möglichst gründlich reinigt und poliert und dabei möglichst wenig schädlichen Abrieb an der natürlichen Zahnhartsubstanz verursacht.

Ein Maß für den Abrieb von Zahnhartsubstanz nach Einsatz einer Reinigungs- und Polierpaste an den Zähnen stellt der sogenannte RDA-Wert (relative dentine abrasion) dar.

Gemessen wurden hier die RDA-Werte gemäß der DIN EN ISO 11609 (Zahnreinigungsmittel, Anforderung, Prüfverfahren und Anforderungen). Bei dieser Meßmethode werden Zähne radioaktiv bestrahlt und anschließend mit den Pasten und mit einem Referenzmittel, basierend auf Kieselsäure, gebürstet. Abschließend wird die verbliebene Radioaktivität gemessen und die Proben mit den Referenzproben verglichen. Die hier angegebenen RDA-Werte sind Mittelwerte aus 6 Probenmessungen. Die Abweichungen werden durch den Variationskoeffizienten (VK) wiedergegeben, einen Wert, der erhalten wird aus dem Quotienten der Standardabweichung und dem Mittelwert multipliziert mit 100.

Aufgabe der vorliegenden Erfindung ist es, dentale Prophylaxepastezusammensetzungen anzugeben, die nicht nur die Zähne der Patienten gründlich reinigen und polieren, sondern insbesondere auch besonders lange Lagerstabilitäten aufweisen und besonders einfach in der Anwendung sind, wobei leicht anzuwendende Polierpasten eine thixotrop standfeste Konsistenz aufweisen sollten.
Die Anwendung der Pasten durch den Zahnarzt erfolgt beispielsweise durch Ausdrücken der Pasten aus Tuben auf eine Vorlage, von der die Paste durch ein rotierendes Applikationsinstrument mit geeigneten Reinigungs- und Polieraufsätzen (Kelche, Bürstchen) aufgenommen wird. Die so mit den Pasten bestückten rotierenden Instrumentenaufsätze werden dann auf die zu behandelnden Zahnoberflächen angelegt, wo die Applikation der Pasten stattfindet.
Eine besonders einfache Anwendung zeichnet sich in einem ersten Schritt dadurch aus, dass die Pasten zum einen leicht, beispielsweise aus den Tuben, ausdrückbar sind, zum anderen aber nach dem Ausdrücken sofort standfest vorliegen, um mit den Applikationsinstrumenten direkt aufgenommen werden zu können. Hierbei ist es besonders wichtig, dass die Paste bei der Aufnahme von der Vorlage auf den Applikator keine Fäden zieht. Die Paste muß in diesem Stadium der Anwendung so beschaffen sein, dass ihre Kohäsionskräfte möglichst gering sind und keine Klebrigkeit auftritt. In einem zweiten Schritt wird die auf dem Applikator vorhandene standfeste Paste auf die zu behandelnden Zahnoberflächen aufgesetzt und mit der Rotation des Applikators begonnen. In diesem Stadium verliert die ehemals standfeste Paste durch das Einwirken von Scherkräften ihre Festigkeit, sie beginnt zu fließen, baut hohe Adhäsionskräfte auf und benetzt jetzt die Oberflächen der zu behandelnden Zähne vollständig.
Unter dem Begriff "thixotrop standfest" verstehen wir somit, dass die Paste von sich aus nicht wegfließt, sie lässt sich gut mit den Applikationswerkzeugen (Kelche, Bürstchen) aufnehmen und in der Mundhöhle anwenden. Die Pasten werden erst dann weicher, wenn während der Anwendung eine Scherbelastung durch rotierende Instrumente auf sie einwirkt. Sie fließen jedoch auch in dieser Situation nicht weg, da sie sehr schnell wieder in den standfesten Zustand zurückkehren, sobald die Rotation nachlässt, bzw. aussetzt.
Diese Eigenschaften verhindern zusätzlich auch ein Verspitzen der Pasten während der Pastenanwendung.
Ein exaktes Maß für die thixotrope Standfestigkeit der Pasten ist der Wert der sogenannten "Spreitbarkeit".
Aus umfangreichen eigenen Untersuchungen ergab sich, dass Pasten mit höheren Spreitbarkeitswerten als 26 mm zu weich sind und nicht mehr das Anforderungsprofil der Eigenschaft "thixotrop standfest" im hier gebrauchten Sinn erfüllen.
Pasten mit niedrigeren Spreitbarkeitswerten als 16 mm erwiesen sich als zu fest. Auch sie erfüllen nicht mehr das Anforderungsaprofil der Eigenschaft "thixotrop standfest" im hier gebrauchten Sinn.
Pasten, die eine Spreitbarkeit von einschließlich 16 mm bis einschließlich 26 mm aufweisen, entsprechen den erfindungsgemäßen Anforderungen an die Konsistenz. Sie erweisen sich als "thixotrop standfest" im hier gebrauchten Sinn und sind somit optimal für eine Prophylaxepaste für den professionellen zahnärztlichen Gebrauch.
Die Bestimmung dieses Wertes wird später im Text genau erläutert.

Unter "besonders lagerstabil" verstehen wir, dass die erfindungsgemäßen Pasten im Gegensatz zu den im Stand der Technik bekannten Pasten auch nach Lagerung bei stark erhöhten Temperaturen von bis zu 50°C sowie unterschiedlichen Luftfeuchtigkeiten (rF) zwischen 10% und 75% und längeren Zeiträumen von bis zu 9 Monaten noch keine Anzeichen von Sedimentation/ Separation aufweisen.
Untersucht wurden Einlagerungen von Prophylaxepasten-zusammensetzungen bei 23°C und 50 % relative Luftfeuchtigkeit (rF), bei 40 °C und 75 % relative Luftfeuchtigkeit und bei 50 °C und 10 % relative Luftfeuchtigkeit.

Ergebnisse der Einlagerungsversuche zur Stabilität am Beispiel einer Prophylaxepaste mit groben Polierkörpern werden unten im Ausführungsteil wiedergegeben.
Aus umfangreichen eigenen Untersuchungen ergab sich, dass ein gutes Maß zur Vorhersage der Stabilitäten nach natürlicher Alterung der Pasten der Wert der sogenannten Zentrifugenstabilität ist.
Zeigen sich im Anschluss an diese Methode in den untersuchten Pasten bei der visuellen Inspektion Anzeichen von Sedimentationen bzw. Separationen, gilt der Test als "nicht bestanden", denn die Homogenität der Phase ist gestört. Liegt nach dem Zentrifugieren eine homogene Paste vor, die keinerlei Anzeichen von Separation bzw. Sedimentation aufweist, dann ist der Test bestanden und die Paste erfindungsgemäß bzw. "in Ordnung" (i.O.). Pasten, die diesen Test bestehen, werden nach natürlicher Einlagerung bei erhöhten Temperaturen und den entsprechenden Luftfeuchtigkeiten auch nach Alterung ebenfalls stabil bleiben.
Die Bestimmung dieses Wertes wird später im Text genau erläutert.

Überraschenderweise zeigte sich, dass besonders lagerstabile und thixotrop standfeste dentale Prophylaxepasten, die zudem sehr einfach in der Anwendung sind und die Zähne der Patienten gründlich reinigen und polieren und wenig Abrieb an natürlicher Zahnsubstanz verursachen, durch eine Pastenzusammensetzung erzielt werden kann, die umfasst
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält.

Solche Prophylaxepasten sind dadurch gekennzeichnet, dass sie besonders lagerstabil, gemessen nach der Methode zur Bestimmung der Zentrifugenstabilität und thixotrop standfest, gemessen nach der Methode zur Bestimmung der Spreitbarkeit, sind.

Diese besonderen Eigenschaften der Prophylaxepasten lassen sich möglicherweise zurückführen auf synergistische Effekte zwischen den Abrasionsstoffen Calciumphosphat und Bimsstein sowie der Verwendung von einem oder mehreren amphoteren Tensiden, dem Puffersystem und der Basiskomponente. Die Wirkung des synergistischen Gemisches auf die Stabilität und dem rheologischen Verhalten der Paste wird durch Verwendung von pyrogenen Kieselsäuren und weiteren Bindemitteln möglicherweise weiter unterstützt.

Erfindungsgemäße Prophylaxepasten weisen somit ein sogenanntes Matrixelement in Form einer Basiskomponente auf, bestehend aus Wasser und dem Feuchthaltemittel. Darin werden dann sukzessive alle in diesem Element löslichen Komponenten wie die Pufferkomponenten, Tensid, gegebenenfalls Cellulosederivate sowie Additive, beispielsweise Natriumfluorid, und Stabilisatoren gelöst und in einem Knettopf gerührt.
Dann werden der Mischung die unlöslichen Komponenten wie Abrasionsmittel, Pigmente sowie Aerosile zugefügt und in einem Kneter zu einer homogenen Masse verarbeitet.
Abschließend wird die Pastenzusammensetzung im Vakuum entlüftet und in Tuben, Näpfchen oder Dosen abgefüllt.

Im Einzelnen können erfindungsgemäße Prophylaxepasten bevorzugt die folgenden Komponenten enthalten:

### Wasser

Erfindungsgemäße Prophylaxepasten sind wässrige Zusammensetzungen. Das Wasser ist bevorzugt aufbereitet, also entmineralisiert, destilliert oder anderweitig gereinigt. Die Menge des eingesetzten Wassers beträgt 15 bis 55 Gew.-%, vorzugsweise 20 bis 45 Gew.-% und ganz bevorzugt 23 bis 35 Gew.-%, wobei die Gewichtsprozentangaben auf die Gesamtmenge der Prophylaxepastenzusammensetzung bezogen sind.

### Feuchthaltemittel

Feuchthaltemittel verhindern das Austrocknen der Pasten und helfen, die Konsistenz der Pasten mitzuregeln und sie stabil zu halten. Auch tragen sie (wie die Tenside) zur Suspensionsvermittlung bei.
In der Regel verwendet man die toxikologisch unbedenklichen Polyole wie Sorbitol, Xylitol, Glycerin, Mannitol, 1,2 Propylenglycol sowie Gemische dieser Verbindungen. Obwohl Xylitol auch ein Süßungsmittel ist, wird diese Verbindung im Rahmen dieser Erfindung den Feuchthaltemitteln zugehörig betrachtet. Weiterhin geeignet sind Polyethylen- oder Polypropylenglykole mit mittleren Molekulargewichten von 200 bis 2000 g/mol.
Erfindungsgemäß bevorzugt ist die Kombination von Glycerin, Sorbitol und Xylitol.

Je nach Produkttyp enthält die Gesamtzusammensetzung der Prophylaxepaste das Feuchthaltemittel oder das Gemisch von Feuchthaltemitteln in einer Menge von 10 bis 60 Gew.-%, vorzugsweise von 17 bis 50 Gew.-% und ganz bevorzugt von 25 bis 42 Gew.-%. Das Verhältnis von Feuchthaltemittel zu Wasser kann 1 : 3 bis 3 : 1 betragen.

### Verdickungsmittel (Komponente zur Steuerung der Rheologie)

Binde- und Verdickungsmittel wirken als Konsistenzregler und steuern die Viskosität und Konsistenz der Pasten. Sie verhindern, im Zusammenwirken mit dem Puffersystem, der Kombination und Menge der Feuchthaltemittel und deren Verhältnis zur Menge des Wassers und Art und Menge des Netzmittels, eine Separation zwischen der flüssigen und festen Phase. Im Allgemeinen werden beispielsweise natürliche und/oder synthetische wasserlösliche Polymere wie Alginate, Stärke und Stärkeether, Celluloseether wie z.B. Carboxymethylcellulose, Hydroxyethylcellulose, Hydroxypropylcellulose, Methylhydroxypropylcellulose, Isopropylcellulose, Methylcellulose sowie Mischungen dieser Derivate, Agar-Agar, Xanthan, Pektine, usw. eingesetzt. Weitere geeignete Mittel sind wasserlösliche Carboxyvinylpolymere, Polyvinylalkohole, Polyvinylpyrrolidone, Polyvinylacetate, Polyvinylpyrrolidon-Vinylacetat Copolymere, Poly(meth)acrylate und Poly(meth)acrylsäuren, vernetzte Poly(meth)acrylate, vernetzte Poly(meth)acrylsäuren, wobei der Begriff (Meth)acrylate sowohl die Acrylate als auch die Methacrylate umfasst, Polyalkylenoxide, mit Maleinsäure modifizierte Polyvinylalkylether, u.s.w. Ebenso können verdickend wirkende pyrogene Kieselsäuren (die nicht zu den KieselsäurePoliermitteln zählen) verwendet werden. Bevorzugt sind diese Kieselsäuren organisch oberflächenmodifiziert.
Erfindungsgemäß bevorzugt ist der Einsatz pyrogener Kieselsäuren und von Cellulosederivaten.
Die Einsatzmengen der Verdickungsmittel in den erfindungsgemäßen Zusammensetzungen betragen 0,1 bis 7 Gew.-%, vorzugsweise 0,1 bis 5 Gew.-% und besonders bevorzugt 0,1 bis 4 Gew.-%.

### Tenside

Tenside werden als oberflächenaktive Benetzungsmittel den Zusammensetzungen beigegeben, um die Kompatibilität der Komponenten untereinander zu verbessern und die Einarbeitung wasserunlöslicher Stoffe zu begünstigen, die Stabilität der Pasten zu erhöhen und zu gewährleisten, dass sich die Pasten während der Anwendung schnell und vollständig am Applikationsort, also auf den zu behandelnden Zahnoberflächen, verteilen und somit auch schwer zugänglichen Stellen, beispielsweise Zahnzwischenräumen, zugänglich sind, so dass sich auch dort die Wirkung der Reinigungs- und der den Pasten zugesetzten Zahnpflegemittel entfalten kann. Tenside unterstützen beispielsweise die Antikarieswirkung von Fluoriden.

Erfindungsgemäß basiert die Prophylaxezusammensetzung auf den ausschließlichen Einsatz eines oder mehrerer amphoterer Tenside. Unter dem Begriff "amphoteres Tensid" werden im Rahmen dieser Erfindung zwitterionische Tenside verstanden, die sowohl eine negativ als auch eine positiv geladene funktionelle Gruppe aufweisen. "Amphotere Tenside" besitzen, wie alle Tenside, einen polaren und einen unpolaren Teil in ihrem Molekülgerüst. Die oberflächenaktiven Verbindungen verfügen im Allgemeinen über eine Alkylgruppe als unpolaren Teil und über mindestens eine quartäre Ammonium-, Phosphonium- oder Sulfoniumgruppe und mindestens eine Carboxylat-, Sulfonat-, Sulfat-, Phosphat- oder Phosphonatgruppe als polaren Teil. Die aliphatische Gruppe kann dabei linear oder verzweigt sein, wobei sie in der Regel zwischen 8 bis 18 Kohlenstoffatome trägt.
Für eine erfindungsgemäße Prophlaxepaste besonders geeignete Tenside sind die sogenannten Betaine, wie die N-Alkyl-N,N-dimethylammoniumglycinate, beispielsweise Trimethylammoniumglycinat, Kokosalkyldimethylammoniumglycinat, N-Acylaminopropyl-N,N,-dimethylammoniumglycinat oder N-Acylaminopropyl-N,N-dimethylammoniumglycinat. Besonders bevorzugt für die Verwendung in einer erfindungsgemäßen Prophylaxepaste ist das unter der Bezeichnung Cocamidopropyl Betaine bekannte Fettsäurederivat, das unter der Bezeichnung "Tego Betain BL 215" kommerziell erhältlich ist.
Ganz besonders bevorzugt für die Verwendung in einer erfindungsgemäßen Prophylaxepaste ist 2-[(3-Dodecanamidopropyl)dimethylamino]acetat. Weitere geeignete Betaine zur Verwendung in einer erfindungsgemäßen Zusammensetzung sind der EP 2 198 836 A1 entnehmbar. Die Einsatzmengen der (des) Netzmittel(s) in den erfindungsgemäßen Zusammensetzungen betragen 0,05 bis 2,6 Gew.-%, vorzugsweise 0,1 bis 2,0 Gew.-% und besonders bevorzugt 0,8 bis 1,2 Gew.-%.

### Pufferkomponenten

Erfindungsgemäß eingesetzt wird ein Puffersystem auf der Basis Natriumdihydrogenphosphat und Dinatriumhydrogenphosphat. Das Puffersystem gewährleistet, daß der pH-Wert der Pasten stabil zwischen pH = 7 und pH = 8 eingestellt bleibt.
Bevorzugt beträgt das Verhältnis der eingesetzten Mengen von Natriumdihydrogenphosphat und Dinatriumhydrogenphosphat in den erfindungsgemäßen Zusammensetzungen 2 : 1 bis 1 :2.

### Putz- und Polierkörper

Putz- und Poliermittel gehören zu den wichtigsten Bestandteilen einer Prophylaxepaste. Sie erledigen die mechanische Entfernung von Zahnbelägen und führen zur Glanzgebung der Zahnoberfläche bei minimaler Scheuerwirkung und möglichst keiner Schädigung des Zahnschmelzes und des Dentins. Das Abrasionsverhalten der Putz- und Polierkörper wird zum großen Teil durch deren Härtegraden, Korngrößenverteilungen und Oberflächenbeschaffenheiten bestimmt. Bei der Auswahl geeigneter Putz- und Polierkörper werden im Allgemeinen solche ausgewählt, die bei hoher Reinigungsleistung und einem hohen Poliereffekt über eine minimale Abrasionswirkung verfügen.
Erfindungsgemäße Putz- und Polierkörper sind beispielsweise Calciumphosphate, Bimsstein, sogenannte Kieselsäure-Poliermittel wie Fällungskieselsäuren, Gelkieselsäuren und Hydrogelkieselsäuren, ferner Silikate wie Aluminiumsilikate und/oder Zirkoniumsilikate, Mischsilikate wie das Natrium-Aluminiumsilikat der Zusammensetzung Na₁₂(AlO₂)₁₂(SiO₂)₁₂ x 7 H₂O, synthetische Zeolithe, Aluminiumoxide, Aluminiumhydroxide, wie Bentonite, Zeolithe, Kaolin, Glimmer, Diatomeenerde, Calciumcarbonat, Magnesiumcarbonat, Trimagnesiumphosphat, Polycarbonate, Siliciumcarbid, Borcarbid, usw., mikrokristalliner Wachs oder auch Kompositharze oder organische Polymere wie Melaminharze, Phenolharze oder Harnstoff-Formaldehydharze, usw. sein. Erfindungsgemäß bevorzugt werden wasserunlösliche anorganische Stoffe verwendet, wobei erfindungswesentlich der Einsatz einer Kombination von wasserunlöslichen Calciumphosphaten, wie beispielsweise der Einsatz des dibasischen Dicalciumorthophosphats CaHPO₄, des monobasischen Monocalciumphosphats Ca(H₂PO₄)₂, des tribasischen Tricalciumphosphats Ca₃(PO₄)₂, des Pentacalciumhydroxytriphosphats (Ca₅(PO₄)₃OH) sowie ihrer Hydrate, weiterhin des Apatits Ca₁₀(PO₄)₆(OH,F,Cl,Br)₂ oder von Ca₈H₂(PO₄)₆ x 5 H₂O ist, wobei eine oder mehrere Verbindungen verwendet werden können und Bimsstein.
Besonders bevorzugt ist der erfindungsgemäße Einsatz einer Kombination von Hydroxylapatit und Bimsstein.
Von mehreren Untersuchten Bimssteinvarianten erwiesen sich diejenigen deren Anteil an Korngrößen kleiner als 45 µm, angegeben als Siebrückstand in %, zwischen 50 und 90% ganz besonders bevorzugt zwischen 70 und 80 % liegt am vorteilhaftesten.

Die Einsatzmenge der Putz- und Polierkörper an der Gesamtzusammensetzung beträgt zwischen 0,5 und 60 Gew.-% und besonders bevorzugt 30 bis 36 Gew.-%. Sie ist, ebenso wie die Auswahl der Mittel und ihr Verhältnis untereinander, auch abhängig von der Pastenart.

Wie oben beschrieben, werden dentale Prophylaxepasten in der Regel in drei unterschiedlichen Polierstärken angeboten: einer weichen, einer mittelharten und einer harten Prophylaxepaste.
In der weichen Polier- und Reinigungspaste liegt das Verhältnis der Menge der Putzkörper der Verbindung auf Basis von Calciumphosphat zu Bimsstein erfindungsgemäß im Bereich von 40:1 und 10:1.
Dabei soll der nach DIN EN ISO 11609 gemessen RDA-Wert erfindungsgemäß in einem Bereich von 5 und 50, vorzugsweise in einem Bereich von 8 und 30 liegen.
In der mittelharten Prophylaxepaste liegt das Verhältnis der Menge der Putzkörper der Verbindung auf Basis von Calciumphosphat zu Bimsstein erfindungsgemäß in einem Bereich von 7 : 1 bis einschließlich 3 :1.

Dabei soll der nach DIN EN ISO 11609 gemessen RDA-Wert erfindungsgemäß vorzugsweise zwischen 70 und 150, vorzugsweise in einem Bereich von 100 und 145 liegen.
In der harten Prophylaxepaste liegt das Verhältnis der Menge der Putzkörper der Verbindung auf Basis von Calciumphosphat zu Bimsstein erfindungsgemäß in einem Bereich ab 3:1 bis 1:1.
Dabei soll der nach DIN EN ISO 11609 gemessen RDA-Wert erfindungsgemäß in einem Bereich von 160 und 250, vorzugsweise in einem Bereich von 170 und 220 liegen.

### Additive

Schließlich können weitere übliche Hilfsmittel zur Verbesserung der Stabilität, der sensorischen und anderer Eigenschaften in den Prophylaxepasten erfindungsgemäß enthalten sein. Solche Additive sind:
- Pigmente wie Titandioxid oder Zinkoxid,
- Farbstoffe
- Konservierungsmittel wie Hydroxybenzoesäuremethyl-, ethyl-, oder propylester, Natriumsorbat oder Natriumbenzoat,
- Fluoridierungsmittel wie Natriumfluorid, Aminfluoride, Kaliumfluorid, Zinnfluorid, Natriummonofluorphosphat oder Zinkfluorid.
Die Einsatzmenge der Additive an der Gesamtzusammensetzung beträgt bevorzugt zwischen 0,001 bis 5 Gew.-%.
Der Einsatz weiterer Hilfsmittel in den erfindungsgemäßen Prophylaxepastenzusammensetzungen ist mit dieser Aufzählung nicht ausgeschlossen.

Besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei der Anteil an Korngrößen des Bimssteins kleiner als 45 µm, angegeben als Siebrückstand in %, zwischen 50 und 90% liegt.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei das Gewichtsverhältnis der beiden Pufferkomponenten Natriumdihydrogenphosphat und Dinatriumhydrogenphosphat untereinander 2 : 1 bis 1:2 beträgt.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Komponente zur Steuerung der Rheologie d.) eine Kombination aus pyrogener Kieselsäure und einem Cellulosederivat ist.

Ebenfalls besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist und wobei der Anteil an Korngrößen des Bimssteins kleiner als 45 µm, angegeben als Siebrückstand in %, zwischen 50 und 90% liegt.

Weiter besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Verbindung auf Basis von Calciumphosphat das Hydroxylapatit ist.

Weiter besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 :2 beträgt und wobei der Anteil an Korngrößen des Bimssteins kleiner als 45 µm, angegeben als Siebrückstand in %, zwischen 50 und 90% liegt.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-% ,
b.) einen Putzkörper im Bereich von 0,5 bis 60 Gew.-% ,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Komponente zur Steuerung der Rheologie d.) eine Kombination aus pyrogener Kieselsäure und einem Cellulosederivat ist und wobei der Anteil an Korngrößen des Bimssteins kleiner als 45 µm, angegeben als Siebrückstand in %, zwischen 50 und 90% liegt.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-% ,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-% ,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt, wobei der Anteil an Korngrößen des Bimssteins kleiner als 45 µm, angegeben als Siebrückstand in %, zwischen 50 und 90% liegt und wobei die Verbindung auf Basis von Calciumphosphat das Hydroxylapatit ist.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist.
Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist und wobei der Anteil an Korngrößen des Bimssteins kleiner als 45 µm, angegeben als Siebrückstand in %, zwischen 50 und 90% liegt.

Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist, wobei der Anteil an Korngrößen des Bimssteins kleiner als 45 µm, angegeben als Siebrückstand in %, zwischen 50 und 90% liegt.
Weitere besonders bevorzugte Ausführungsformen erfindungsgemäßer Prophylaxepasten für den professionellen zahnärztlichen Gebrauch umfassen
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich von 0,5 bis 60 Gew.-%,
c.) eine Pufferkomponente,
d.) eine Komponente zur Steuerung der Rheologie im Bereich von 0,1 bis 7 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist, wobei der Anteil an Korngrößen des Bimssteins kleiner als 45 µm, angegeben als Siebrückstand in %, zwischen 50 und 90% liegt.

Ebenfalls besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei das Gewichtsverhältnis der beiden Pufferkomponenten Natriumdihydrogenphosphat und Dinatriumhydrogenphosphat untereinander 2 : 1 bis 1 : 2 beträgt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Komponente zur Steuerung der Rheologie d.) eine Kombination aus pyrogener Kieselsäure und einem Cellulosederivat ist.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Verbindung auf Basis von Calciumphosphat das Hydroxylapatit ist.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei die Verbindung auf Basis von Calciumphosphat das Hydroxylapatit ist und wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei die Komponente zur Steuerung der Rheologie d.) eine Kombination aus pyrogener Kieselsäure und einem Cellulosederivat ist und wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt, wobei die Komponente zur Steuerung der Rheologie d.) eine Kombination aus pyrogener Kieselsäure und einem Cellulosederivat ist.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt, wobei die Verbindung auf Basis von Calciumphosphat das Hydroxylapatit ist und wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist, wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist, wobei der Anteil an Korngrößen des Bimssteins kleiner als 45 µm, angegeben als Siebrückstand in %, zwischen 50 und 90% liegt.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist.

Weiterhin besonders bevorzugte Ausführungsformen erfindungsgemäßer Prophylaxepasten für den professionellen zahnärztlichen Gebrauch enthalten
a.) eine Basiskomponente, enthaltend Wasser vorzugsweise im Bereich von 20 bis 45 Gew.-% und Feuchthaltemittel vorzugsweise im Bereich von 17 bis 50 Gew.-%,
b.) einen Putzkörper im Bereich von 0,5 bis 60 Gew.-%,
c.) eine Pufferkomponente,
d.) eine Komponente zur Steuerung der Rheologie vorzugsweise im Bereich von 0,1 bis 5 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge vorzugsweise im Bereich von 0,1 bis 2,0 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei das Gewichtsverhältnis der beiden Pufferkomponenten Natriumdihydrogenphosphat und Dinatriumhydrogenphosphat untereinander 2 : 1 bis 1 : 2 beträgt und wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist, wobei der Anteil an Korngrößen des Bimssteins kleiner als 45 µm, angegeben als Siebrückstand in %, zwischen 50 und 90% liegt.

Ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält.

Weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist.

Weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei der Anteil an Korngrößen des Bimssteins kleiner als 45 µm, angegeben als Siebrückstand in %, zwischen 70 und 80% liegt.

Weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält.

Weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Komponente zur Steuerung der Rheologie d.) eine Kombination aus pyrogener Kieselsäure und einem Cellulosederivat ist.

Weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist und wobei wobei der Anteil an Korngrößen des Bimssteins kleiner als 45 µm, angegeben als Siebrückstand in %, zwischen 70 und 80% liegt.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält und wobei die Komponente zur Steuerung der Rheologie d.) eine Kombination aus pyrogener Kieselsäure und einem Cellulosederivat ist, wobei der Anteil an Korngrößen des Bimssteins kleiner als 45 µm, angegeben als Siebrückstand in %, zwischen 70 und 80% liegt.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält, wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält, wobei die Komponente zur Steuerung der Rheologie d.) eine Kombination aus pyrogener Kieselsäure und einem Cellulosederivat ist.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält und wobei der Anteil an Korngrößen des Bimssteins kleiner als 45 µm, angegeben als Siebrückstand in %, zwischen 70 und 80% liegt.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält und wobei die Verbindung auf Basis von Calciumphosphat das Hydroxylapatit ist und wobei der Anteil an Korngrößen des Bimssteins kleiner als 45 µm, angegeben als Siebrückstand in %, zwischen 70 und 80% liegt.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist und wobei der Anteil an Korngrößen des Bimssteins kleiner als 45 µm, angegeben als Siebrückstand in %, zwischen 70 und 80% liegt.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält wobei die Komponente zur Steuerung der Rheologie d.) eine Kombination aus pyrogener Kieselsäure und einem Cellulosederivat ist und wobei der Anteil an Korngrößen des Bimssteins kleiner als 45 µm, angegeben als Siebrückstand in %, zwischen 70 und 80% liegt.

Noch weitere ganz besonders bevorzugte Ausführungsformen erfindungsgemäßer Prophylaxepasten für den professionellen zahnärztlichen Gebrauch enthalten
a.) eine Basiskomponente, enthaltend Wasser im Bereich von 23 bis 35 Gew.-% und Feuchthaltemittel im Bereich von 25 bis 42 Gew.-%,
b.) einen Putzkörper im Bereich von 30 bis 36 Gew.-%,
c.) eine Pufferkomponente,
d.) eine Komponente zur Steuerung der Rheologie im Bereich von 0,1 bis 4 Gew.-%
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel, wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,8 bis 1,2 Gew.-% aufweist,
wobei sich alle Gew.-% auf die Gesamtmasse der Prophylaxepaste beziehen und wobei die Pufferkomponente c.) auf Hydrogenphosphatbasis basiert und das Gewichtsverhältnis der beiden Pufferkomponenten Natriumdihydrogenphosphat und Dinatriumhydrogenphosphat untereinander 2 : 1 bis 1 : 2 beträgt und wobei das Puffersystem den pH-Wert der Prophylaxepaste, ermittelt in einer 10 %igen Suspension (w/w) der Paste in Wasser gegen eine pH-Elektrode, zwischen 7 und 8 hält wobei die Komponente zur Steuerung der Rheologie d.) eine Kombination aus pyrogener Kieselsäure und einem Cellulosederivat ist und die Verbindung auf Basis von Calciumphosphat das Hydroxylapatit ist und wobei der Anteil an Korngrößen des Bimssteins kleiner als 45 µm, angegeben als Siebrückstand in %, zwischen 70 und 80% liegt.

Die Erfindung betrifft ferner ein Kit, umfassend Tuben, Näpfchen oder Dosen enthaltend erfindungsgemäße weiche und/oder erfindungsgemäße mittelharte und/oder erfindungsgemäße harte Prophylaxepasten sowie gegebenenfalls Reinigungs- und/oder Polierkelche und/oder Polierbürstchen und/oder Fluoridierungsmittel und/oder Bleichmittel und/oder Remineralisierungsmittel und/oder (Fissuren)versiegler.

### Ausführungsbeispiele der Erfindung

### Bestimmung des pH-Werts

Für die Ermittlung des pH-Werts werden 2 g Substanz in 20 ml demineralisiertem Wasser suspendiert. Anschließend erfolgt die Messung des pH-Werts gegen eine pH-Elektrode.

### Bestimmung der Spreitbarkeit

Die Bestimmung der Spreitbarkeit erfolgt im Klimaraum bei einer Temperatur von 23 °C und einer relativen Luftfeuchtigkeit von 50% mittels einer Kunststoffglasplattenanordnung. Die Dimensionen der Platten betragen 60 x 60 x 5 mm bei einem Plattengewicht von 25 g. Bei der Bestimmung werden auf die erste Kunststoffglasplatte 0,5 ml der jeweiligen Paste mittels einer Spritze in der Mitte der Platte platziert. Anschließend wird die zweite Kunststoffglasplatte parallel zur ersten aufgelegt und mittig mit einem 100 g schweren zylinderförmigen Eisenstempel (Durchmesser: 33 mm, Höhe: 13 mm) 10 Minuten belastet.
Nach der Belastung wird der Mittelwert von zwei Durchmessern des entstandenen Probenzylinders bestimmt.
Diese Methode ist ein gängiges Verfahren zur Bestimmung der Konsistenz bei der Herstellung von Pasten.

### Bestimmung der Zentrifugenstabilität

Zur Beurteilung der Zentrifugenstabilität wird in einer Labor-Standard-"EBA 20" Zentrifuge von Hettich 3g der jeweiligen Paste in ein Zentrifugenglas, das einen Durchmesser von 15 mm sowie eine Länge von 100 mm hat, eingewogen. Anschließend wird die Paste bei 5000 U/min 15 Minuten zentrifugiert. Die Temperatur während des Zentrifugenlaufs beträgt 23°C.

### RDA-Werte

Die RDA-Werte wurden, wie oben beschrieben, gemäß der DIN EN ISO 11609 ermittelt. Es ergaben sich die folgenden Werte:

| | |
|---|---|
| Weiche Paste | RDA-Wert 16 (VK (%) 4,9) |
| Mittelharte Paste | RDA-Wert 127 (VK (%) 10,1) |
| Harte Paste | RDA-Wert 195 (VK (%) 2,4) |

Die nachfolgenden Beispiele dienen der Veranschaulichung der vorliegenden Erfindung, schränken diese jedoch in keiner Weise ein.

Die Herstellung der Prophylaxepasten in den unten dargestellten Beispielen für verschiedene Variationen ist wie folgt:
In ein Matrixelement bestehend aus Wasser und dem Feuchthaltemittel werden sukzessive alle in diesem Element löslichen Komponenten, die Pufferkomponenten, Tensid, gegebenenfalls Cellulosederivate sowie Additive, beispielsweise Natriumfluorid und Stabilisatoren gelöst und in einem Knettopf gerührt. Anschließend werden der Mischung die unlöslichen Komponenten wie Abrasionsmittel, Pigmente sowie Aerosile zugefügt und in einem Kneter zu einer homogenen Masse verarbeitet.

Abschließend wird die Pastenzusammensetzung im Vakuum entlüftet und in Tuben, Näpfchen oder Dosen abgefüllt

### Beispiel 1

Am Beispiel von unterschiedlichen weichen Prophylaxepasten soll der Einfluss verschiedener Tensidtypen auf die physikalischen Eigenschaften veranschaulicht werden. Hierbei werden identische Pastenformulierungen verwendet, die sich nur im Tensidtyp (amphoter, anionisch, kationisch,) unterscheiden.

| **Inhaltsstoffe** | **m%** | **m%** | **m%** |
|---|---|---|---|
| Wasser | 27,00 | 27,00 | 27,00 |
| Glycerin (85%) | 10,36 | 10,36 | 10,36 |
| Sorbitol (70%) | 17,61 | 17,61 | 17,61 |
| Methylcellulose | 1,04 | 1,04 | 1,04 |
| Xylitol | 3,11 | 3,11 | 3,11 |
| Natriumfluorid | 0,15 | 0,15 | 0,15 |
| Stabilisatoren (Paraben) | 0,21 | 0,21 | 0,21 |
| Natirumdihydrogenphophat | 1,04 | 1,04 | 1,04 |
| Dinatriumhydropenphosphat | 1,04 | 1,04 | 1,04 |
| Calciumphosphat | 33,12 | 33,12 | 33,12 |
| Bimsstein | 2,00 | 2,00 | 2,00 |
| Titandioxid | 1,04 | 1,04 | 1,04 |
| Pyrogene Kieselsäure (<30nm) | 1,24 | 1,24 | 1,24 |
| (1-Dodecyl)trimethylammoniumchlorid | 0,00 | 0,00 | **1,04** |
| Laurylsulfat | 0,00 | **1,04** | 0,00 |
| Betain | **1,04** | 0,00 | 0,00 |
| **Summe** | **100,0** | **100,0** | **100,0** |

| pH-Wert | 7,34 | 7,33 | 7,27 |
|---|---|---|---|
| Zentrifugenstabilität | i.O. | i.O. | nicht bestanden |
| Spreitbarkeit | 18,0 | 27 | 29 |
| Aussehen | Gut | Kaum thixotrop | Inhomogen |

Überraschenderweise zeigt sich, dass die Verwendung von anionischen bzw. kationischen Tensiden zu deutlich unterschiedlichen Eigenschaften gegenüber den erfindungsgemäßen Pasten führt.
Die mit einem anionischen Tensid formulierte Prophylaxepaste ist äußerst weich und zeigt nur ein sehr schwach ausgeprägtes thixotrop standfestes Verhalten entsprechend dem Wert der Spreitbarkeit. Zusatzversuche zeigten zudem, dass eine gute Benetzung der Zahnoberfläche mit dieser Zusammensetzung nicht erreicht werden kann.
Bei Verwendung eines kationischen Tensides wird eine in sich inhomogene, ebenfalls sehr weiche Prophylaxepaste erhalten. In der Zentrifuge und während der Lagerung kommt es sehr schnell zu einer Sedimentation der Putzkörper. Eine erfolgreiche zahnprophylaktische Anwendung ist mit einem solchen Pastensystem nicht möglich.
Demgegenüber steht die erfindungsgemäße Prophylaxepaste mit einem amphoteren Tensid, die sich durch ihr thixotrop standfestes Verhalten bei gleichzeitig guter Lagerstabilität auszeichnet.

### Beispiel 2

Am Beispiel verschiedener harter Prophylaxepasten soll erläutert werden, welchen Einfluss unterschiedlich prozentuale Anteile an amphoterem Tensid in Prophylaxepasten haben.

| **Inhaltsstoffe** | **m%** | **m%** | **m%** | **m%** | **m%** |
|---|---|---|---|---|---|
| Wasser | 26,78 | 27,06 | 26,92 | 26,51 | 26,24 |
| Glycerin (85%) | 16,28 | 16,45 | 16,36 | 16,11 | 15,95 |
| Sorbitol (70%) | 12,36 | 12,49 | 12,43 | 12,24 | 12,11 |
| Methylcellulose | 1,03 | 1,04 | 1,04 | 1,02 | 1,01 |
| Xylitol | 5,15 | 5,20 | 5,18 | 5,10 | 5,05 |
| Natriumfluorid | 0,14 | 0,15 | 0,14 | 0,14 | 0,14 |
| Stabilisatoren (Paraben) | 0,21 | 0,21 | 0,21 | 0,20 | 0,20 |
| Natriumdihydrogenphosphat | 1,03 | 1,04 | 1,04 | 1,02 | 1,01 |
| Dinatriumhydrogenphosphat | 1,03 | 1,04 | 1,04 | 1,02 | 1,01 |
| Pyrogene Kieselsäure (<30nm) | 1,99 | 2,01 | 2,00 | 1,97 | 1,95 |
| Titandioxid | 1,03 | 1,04 | 1,04 | 1,02 | 1,01 |
| Calciumphosphat | 16,48 | 16,65 | 16,57 | 16,31 | 16,15 |
| Bimsstein | 15,45 | 15,62 | 15,51 | 15,30 | 15,14 |
| **Betain** | 1,04 | 0,00 | 0,52 | 2,04 | 3,03 |
| **Summe** | **100,0** | **100,0** | **100,0** | **100,0** | **100,0** |

| pH-Wert | 7.22 | 7.28 | 7.28 | 7.24 | 7.23 |
|---|---|---|---|---|---|
| Zentrifugenstabilität | i.O. | Nicht bestanden | i.O. | i.O. | Nicht bestanden |
| Spreitbarkeit | 20 | 27 | 20.5 | 22 | 29.5 |
| Aussehen | Gut | Sehr Weich & Separation | Gut | Gut | Sehr weich & Separation |

Es zeigt sich, dass ein zu hoher bzw. zu niedriger Anteil an amphoterem Tensid zu einer sehr weichen Konsistenz führt welches sich in den Werten der Spreitbarkeit wiederspiegelt. Zusatzversuche zeigten zudem, dass eine gute Benetzung von Zahnoberflächen mit diesen Zusammensetzungen nicht erreicht werden können.

In der Zentrifuge und während der Lagerung kommt es sehr schnell zu einer Sedimentation der Putzkörper. Eine erfolgreiche zahnprophylaktische Anwendung ist mit einem solchen Pastensystem nicht möglich.

### Beispiel 3

Am Beispiel unterschiedlicher Prophylaxepasten (weich, mittelhart, hart) soll der Einfluss des pH-Werts auf die Eigenschaften der Pasten verdeutlicht werden. Zur Variation des pH-Werts in den Pasten wird das Dinatriumhydrogenphosphat konstant gehalten und der Gehalt an saurem Natriumdihydrogenphosphat variiert.

**Versuchsreihe 1: Weiche Pasten**

| **Inhaltsstoffe** | **m%** | **m%** | **m%** | **m%** | **m%** |
|---|---|---|---|---|---|
| Wasser | 27,00 | 27,31 | 27,15 | 26,71 | 26,41 |
| Glycerin (85%) | 10,36 | 10,47 | 10,41 | 10,25 | 10,15 |
| Sorbitol (70%) | 17,61 | 17,79 | 17,70 | 17,43 | 17,25 |
| Methylcellulose | 1,04 | 1,05 | 1,04 | 1,03 | 1,01 |
| Xylitol | 3,11 | 3,14 | 3,12 | 3,08 | 3,04 |
| Natriumfluorid | 0,15 | 0,15 | 0,15 | 0,14 | 0,14 |
| Stabilisatoren (Paraben) | 0,21 | 0,21 | 0,21 | 0,21 | 0,20 |
| **Natriumdihydrogenphosphat** | 1,04 | 0,00 | 0,53 | 2,05 | 3,04 |
| Dinatriumhydrogenphosphat | 1,04 | 1,03 | 1,04 | 1,03 | 1,01 |
| Calciumphosphat | 33,12 | 33,49 | 33,32 | 32,78 | 32,47 |
| Bimsstein | 2,00 | 2,00 | 2,00 | 2,00 | 2,00 |
| Titandioxid | 1,04 | 1,05 | 1,04 | 1,03 | 1,01 |
| Pyrogene Kieselsäure (<30nm) | 1,24 | 1,26 | 1,25 | 1,23 | 1,22 |
| Betain | 1,04 | 1,05 | 1,04 | 1,03 | 1,05 |
| **Summe** | 100,0 | 100,0 | 100,0 | 100,0 | 100,0 |
| | | | | | |

| pH-Wert | 7,34 | 8,27 | 7,68 | 7,04 | 6,66 |
|---|---|---|---|---|---|
| Zentrifugenstabilität | i.O. | Nicht bestanden | i.O. | i.O. | i.O. |
| Spreitbarkeit | 18,5 | 27 | 19,5 | 16 | 14 |
| Aussehen | Gut | Weich & cremig | Gut | Gut | sehr fest & trocken |

**Versuchsreihe 2 : Mittelharte Pasten**

| **Inhaltsstoffe** | **m%** | **m%** | **m%** | **m%** | **m%** |
|---|---|---|---|---|---|
| Wasser | 24,20 | 24,46 | 24,33 | 23,96 | 23,72 |
| Glycerin (85%) | 16,14 | 16,31 | 16,22 | 15,97 | 15,82 |
| Sorbitol (70%) | 6,05 | 6,11 | 6,08 | 5,99 | 5,93 |
| Methylcellulose | 1,01 | 1,02 | 1,01 | 1,00 | 0,99 |
| Xylitol | 14,12 | 14,27 | 14,19 | 13,98 | 13,84 |
| Natriumfluorid | 0,14 | 0,14 | 0,14 | 0,14 | 0,14 |
| Stabilisatoren (Paraben) | 0,20 | 0,20 | 0,20 | 0,20 | 0,20 |
| **Natriumdihydrogenphosphat** | 1,01 | 0,00 | 0,51 | 2,00 | 2,97 |
| Dinatriumhydrogenphosphat | 1,01 | 1,02 | 1,03 | 1,00 | 0,99 |
| Pyrogene Kieselsäure (<30nm) | 1,84 | 1,85 | 1,84 | 1,82 | 1,80 |
| Titandioxid | 1,01 | 1,02 | 1,01 | 1,00 | 0,99 |
| Calciumphosphat | 24,22 | 24,50 | 24,35 | 23,95 | 23,70 |
| Bimsstein | 8,04 | 8,08 | 8,08 | 7,99 | 7,92 |
| Betain | 1,01 | 1,02 | 1,01 | 1,00 | 0,99 |
| **Summe** | **100,0** | **100,0** | **100,0** | **100,0** | **100,0** |
| | | | | | |

| pH-Wert | 7,28 | 8,2 | 7,64 | 7,03 | 6,60 |
|---|---|---|---|---|---|
| Zentrifugenstabilität | i.O. | Nicht bestanden | i.O. | i.O. | i.O. |
| Spreitbarkeit | 19 | 27 | 21 | 16 | 14 |
| Aussehen | Gut | Weich & Separation | Gut | Gut | Fest & Trocken |

**Versuchsreihe 3: Harte Pasten**

| **Inhaltsstoffe** | **m%** | **m%** | **m%** | **m%** | **m%** |
|---|---|---|---|---|---|
| Wasser | 26,78 | 27,06 | 26,92 | 26,51 | 26,24 |
| Glycerin (85%) | 16,28 | 16,45 | 16,36 | 16,11 | 15,95 |
| Sorbitol (70%) | 12,36 | 12,49 | 12,43 | 12,24 | 12,11 |
| Methylcellulose | 1,03 | 1,04 | 1,04 | 1,02 | 1,01 |
| Xylitol | 5,15 | 5,20 | 5,18 | 5,10 | 5,05 |
| Natriumfluorid | 0,14 | 0,15 | 0,14 | 0,14 | 0,14 |
| Stabilisatoren (Paraben) | 0,21 | 0,21 | 0,21 | 0,20 | 0,20 |
| **Natriumdihydrogenphosphat** | 1,03 | 0,00 | 0,52 | 2,04 | 3,03 |
| Dinatriumhydrogenphosphat | 1,03 | 1,04 | 1,04 | 1,02 | 1,01 |
| Pyrogene Kieselsäure (<30nm) | 1,99 | 2,01 | 2,00 | 1,97 | 1,95 |
| Titandioxid | 1,03 | 1,04 | 1,04 | 1,02 | 1,01 |
| Calciumphosphat | 16,48 | 16,65 | 16,57 | 16,31 | 16,15 |
| Bimsstein | 15,45 | 15,62 | 15,51 | 15,30 | 15,14 |
| Betain | 1,04 | 1,04 | 1,04 | 1,02 | 1,01 |
| **Summe** | **100,0** | **100,0** | **100,0** | **100,0** | **100,0** |
| | | | | | |

| pH-Wert | 7,26 | 8,39 | 7,67 | 7,04 | 6,65 |
|---|---|---|---|---|---|
| Zentrifugenstabilität | i.O. | Nicht bestanden | i.O. | i.O. | i.O. |
| Spreitbarkeit | 21 | 27 | 22 | 16 | 13 |
| Aussehen | Gut | Weich & Separation | Gut | Gut | Fest & Trocken |

Die Variation des sauren Mononatriumdihydrogenphosphat führt zu einer deutlichen pH-Wert Schwankung in den verschieden Pasten.
Es zeigt sich, dass im basischen Bereich oberhalb von pH=8 eine sehr dünne Konsistenz vorliegt und die Pasten sehr schnell separieren, während im sauren Bereich unterhalb von pH=7 die Pasten sehr fest und trocken werden. Diese Pasten entsprechen nicht mehr dem erfindungsgemäßen Anforderungsprofil.
Die Versuche verdeutlichen, dass der optimale pH-Bereich für die Pasten zwischen 7 und 8 liegt. Über diesem Bereich, im stärker Basischen bzw. unterhalb von diesem Bereich im Sauren, haben die Pasten nicht mehr die erfindungsgemäßen Eigenschaften. Der optimale pH-Bereich kann durch eine Kombination von unterschiedlichen Mengen an Mononatriumdihydrogenphosphat und Dinatriumdihydrogenphosphat genau eingestellt werden.

### Beispiel 4

Am Beispiel einer harten Prophylaxepaste soll der Einfluss der Temperatur bzw. Luftfeuchtigkeit auf die Eigenschaften einer erfindungsgemäßen harten Paste über einen Zeitraum von 9 Monaten dargelegt werden. Die Paste wurde bei 23°C, 50% rF, 40°C, 75% rF und 50°C, 10% rF untersucht.

**Verwendete harte Paste:**

| **Inhaltsstoffe** | **m%** |
|---|---|
| Wasser | 26,78 |
| Glycerin (85%) | 16,28 |
| Sorbitol (70%) | 12,36 |
| Methylcellulose | 1,03 |
| Xylitol | 5,15 |
| Natriumfluorid | 0,14 |
| Stabilisatoren (Paraben) | 0,21 |
| **Natriumdihydrogenphosphat** | 1,03 |
| Dinatriumhydrogenphosphat | 1,03 |
| Pyrogene Kieselsäure (>30nm) | 1,99 |
| Titandioxid | 1,03 |
| Calciumphosphat | 16,48 |
| Bimsstein | 15,45 |
| Betain | 1,04 |
| **Summe** | **100,0** |

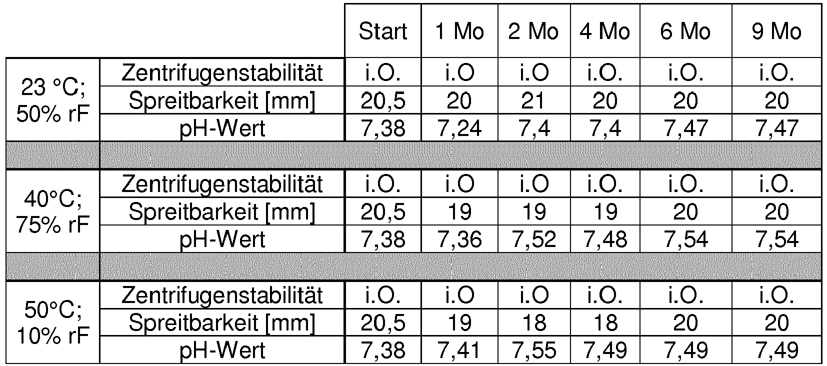

Der Versuch zeigt, dass die erfindungsgemäße Prophylaxepaste auch nach Lagerung bei stark erhöhten Temperaturen von bis zu 50°C sowie unterschiedlichen Luftfeuchtigkeiten (rF) zwischen 10% und 75% und längeren Zeiträumen bis zu 9 Monaten noch keine Anzeichen von Sedimentation/ Separation aufweisen. Auch die erfindungsgemäßen thixotrop standfesten Eigenschaften bleiben über den gesamten Zeitraum erhalten. Prophylaxepasten aus dem Stand der Technik weisen diese Eigenschaften nicht auf.

## Patentansprüche

1. Prophylaxepaste für den professionellen zahnärztlichen Gebrauch, umfassend
a.) eine Basiskomponente, enthaltend Wasser im Bereich von 15 bis 55 Gew.-% und Feuchthaltemittel im Bereich von 10 bis 60 Gew.-%,
b.) einen Putzkörper im Bereich 0,5 bis 60 Gew.-%,
c.) eine Pufferkomponente,
d.) eine Komponente zur Steuerung der Rheologie im Bereich von 0,1 bis 7 Gew.-% ,
e.) eine Additivkomponente im Bereich von 0,001 bis 5 Gew.-%, wobei die Additive ausgewählt sind aus einer Liste umfassend Stabilisatoren, Farbstoffe, Pigmente und Fluoridierungsmittel,
wobei die Prophylaxepaste als Putzkörper b.) eine Verbindung auf der Basis von Calciumphosphat in Kombination mit Bimsstein enthält und als Netzmittel
f.) ausschließlich ein oder mehrere amphotere(s) Tensid(e) in einer Menge im Bereich von 0,05 bis 2,6 Gew.-% aufweist,
wobei sich alle Gewichtsprozente auf die Gesamtmasse der Prophylaxepaste beziehen und
wobei die Pufferkomponente c.) auf Hydrogenphosphatbasis basiert und den pH-Wert der Prophylaxepaste, ermittelt in einer 10 %igen Suspension (w/w) der Paste in Wasser gegen eine pH-Elektrode, zwischen 7 und 8 hält.

2. Prophylaxepaste für den professionellen zahnärztlichen Gebrauch nach Anspruch 1, wobei die Verbindung auf Basis von Calciumphosphat das Hydroxylapatit ist.

3. Prophylaxepaste für den professionellen zahnärztlichen Gebrauch nach Anspruch 1 oder 2, wobei der Anteil an Korngrößen des Bimssteins kleiner als 45 µm, angegeben als Siebrückstand in %, zwischen 50 und 90% vorzugsweise zwischen 70 und 80% liegt.

4. Prophylaxepaste für den professionellen zahnärztlichen Gebrauch nach einem der vorhergehenden Ansprüche, wobei das Gewichtsverhältnis der beiden Pufferkomponenten Natriumdihydrogenphosphat und Dinatriumhydrogenphosphat untereinander 2 : 1 bis 1 : 2 beträgt.

5. Prophylaxepaste für den professionellen zahnärztlichen Gebrauch nach einem der vorhergehenden Ansprüche, wobei die Komponente zur Steuerung der Rheologie d.) eine Kombination aus pyrogener Kieselsäure und einem Cellulosederivat ist.

6. Prophylaxepaste für den professionellen zahnärztlichen Gebrauch nach einem der vorangehenden Ansprüche, wobei die Paste umfaßt
- Wasser im Bereich von 20 bis 45 Gew.-% und Feuchthaltemittel im Bereich von 17 bis 50 Gew.-%
- eine Komponente zur Steuerung der Rheologie im Bereich von 0,1 bis 5 Gew.-%,
- ausschließlich ein oder mehrere amphotere(s) Tensid(e) in einer Menge im Bereich von 0,1 bis 2,0 Gew.-%,
wobei sich alle Gew.-% auf die Gesamtmasse der Prophylaxepaste beziehen.

7. Prophylaxepaste für den professionellen zahnärztlichen Gebrauch nach einem der Ansprüche 1-5, wobei die Paste umfaßt
- Wasser im Bereich von 23 bis 35 Gew.-% und Feuchthaltemittel im Bereich von 25 bis 42 Gew.-%,
- einen Putzkörper im Bereich von 30 bis 36 Gew.-%,
- eine Komponente zur Steuerung der Rheologie im Bereich von 0,1 bis 4 Gew.-%,
- ausschließlich ein oder mehrere amphotere(s) Tensid(e) in einer Menge im Bereich von 0,80 bis 1,20 Gew.-%,
wobei sich alle Gew.-% auf die Gesamtmasse der Prophylaxepaste beziehen.

8. Prophylaxepaste für den professionellen zahnärztlichen Gebrauch nach einem der Ansprüche 1-7, **dadurch gekennzeichnet, dass** sie lagerstabil, gemessen nach der Methode zur Bestimmung der Zentrifugenstabilität, wobei die Bestimmung der Zentrifugenstabilität mit einer Labor-Standard-Zentrifuge (EBA 20® von Hettich) erfolgt und wobei 3 g der jeweiligen Paste in ein Zentrifugenglas, das einen Durchmesser von 15 mm und eine Länge von 100 mm aufweist, eingewogen und anschließend bei 5000 U/min 15 Minuten zentrifugiert wird, wobei die Temperatur während des Zentrifugenlaufs 23°C beträgt und wobei im Anschluß an diese Methode die untersuchten Pasten visuell auf Phasenhomogenität untersucht werden und thixotrop standfest, gemessen nach der Methode zur Bestimmung der Spreitbarkeit, wobei die Bestimmung der Spreitbarkeit in einem Klimaraum bei einer Temperatur von 23 °C und einer relativen Luftfeuchtigkeit von 50% mittels einer Kunststoffglasplattenanordnung erfolgt, wobei die Dimensionen der Platten 60 x 60 x 5 mm betragen und wobei das Plattengewicht 25 g beträgt und wobei mittels einer Spritze 0,5 ml der jeweiligen Paste auf die erste Kunststoffglasplatte in der Mitte der Platte platziert wird und wobei anschließend die zweite Kunststoffglasplatte parallel zur ersten aufgelegt und mittig mit einem 100 g schweren zylinderförmigen Eisenstempel mit einem Durchmesser von 33 mm und einer Höhe von 13 mm für 10 Minuten belastet wird und wobei der Wert der Spreitbarkeit als Mittelwert von zwei Durchmessern des entstandenen Probenzylinders nach der Belastung bestimmt wird, ist.

9. Kit, umfassend Tuben, Näpfchen oder Dosen enthaltend eine oder mehrere weiche Prophylaxepaste(n) nach einem der vorangehenden Ansprüche, **dadurch gekennzeichnet, dass** das Verhältnis der Putzkörper der Verbindung auf Basis von Calciumphosphat zu Bimsstein in einem Bereich von 40 : 1 und 10:1 liegt und sich der nach DIN EN ISO 11609 gemessene RDA - Wert der weichen Paste(n) im Bereich von 5 und 50, vorzugsweise im Bereich von 8 und 30 befindet und/oder eine oder mehrere mittelharte Prophylaxepaste(n) nach einem der vorangehenden Ansprüche, **dadurch gekennzeichnet, dass** das Verhältnis der Putzkörper der Verbindung auf Basis von Calciumphosphat zu Bimsstein in einem Bereich von 7 : 1 bis einschließlich 3 : 1 liegt und sich der nach DIN EN ISO 11609 gemessene RDA - Wert der mittelharten Paste(n) im Bereich von 70 und 150, vorzugsweise im Bereich von 100 und 145 befindet, und/oder eine oder mehrere harte Prophylaxepaste(n) nach einem der vorangehenden Ansprüche, **dadurch gekennzeichnet, dass** das Verhältnis der Putzkörper der Verbindung auf Basis von Calciumphosphat zu Bimsstein in einem Bereich ab 3 : 1 bis 1 : 1 liegt und sich der nach DIN EN ISO 11609 gemessene RDA - Wert der harten Paste(n) im Bereich von 160 und 250, vorzugsweise im Bereich von 170 und 220 befindet sowie gegebenenfalls Reinigungs- und/oder Polierkelche und/oder Polierbürstchen und/oder Fluoridierungsmittel und/oder Bleichmittel und/oder Remineralisierungsmittel und/oder (Fissuren)-versiegler.

10. Verfahren zur Herstellung einer Prophylaxepaste nach einem der Ansprüche 1 bis 8 mit folgenden Schritten:
a.) Bereitstellen der Basiskomponente, bestehend aus Wasser und Feuchthaltemittel,
b.) Sukzessives Lösen aller weiteren, in dieser Komponente löslichen Bestandteile der Prophylaxepaste,
c.) Zuführung der in dieser Mischung unlöslichen Bestandteile,
d.) Verarbeitung der Mischung zu einer homogenen Masse,
e.) Entlüftung der Prophylaxepastenzusammensetzung im Vakuum und
f.) Abfüllung der Prophylaxepaste in Tuben, Näpfchen oder Dosen.

11. Verwendung einer Zusammensetzung nach den Ansprüchen 1 bis 8 zur Herstellung einer Prophylaxepaste.

## Claims

1. Prophylaxis paste for professional dental use, comprising
a.) a base component comprising water in the range from 15 to 55% by weight, and humectant in the range from 10 to 60% by weight,
b.) a cleaning body in the range from 0,5 to 60% by weight,
c.) a buffer component,
d.) a component for controlling the rheology in the range from 0,1 to 7% by weight,
e.) an additive component in the range from 0,001 to 5% by weight, wherein the additives are selected from a group consisting of stabilizers, dyes, pigments and fluoridation agents,
wherein the prophylaxis paste comprises, as cleaning body b.), a compound based on calcium phosphate in combination with pumice and, as wetting agent,
f.) has exclusively one or more amphoteric surfactant(s) in an amount in the range of 0,05 to 2,6% by weight, wherein all of the percents by weight refer to the total mass of the prophylaxis paste and
wherein the buffer component c.) is based on hydrogen phosphate and keeps the pH of the prophylaxis paste, measured in a 10% suspension (w/w) of the paste in water against a pH electrode, between 7 and 8.

2. Prophylaxis paste for professional dental use according to Claim 1, wherein the compound based on calcium phosphate is hydroxylapatite.

3. Prophylaxis paste for professional dental use according to Claim 1 or 2, wherein the fraction of particle sizes of the pumice of less than 45 µm, stated as sieve residue in %, is between 50 and 90%, preferably between 70 and 80%.

4. Prophylaxis paste for professional dental use according to one of the preceding claims, wherein the weight ratio of the two buffer components sodium dihydrogen phosphate and disodium hydrogen phosphate with one another is 2:1 to 1:2.

5. Prophylaxis paste for professional dental use according to one of the preceding claims, wherein the component for controlling the rheology d.) is a combination of fumed silica and a cellulose derivative.

6. Prophylaxis paste for professional dental use according to one of the preceding claims, wherein the paste comprises
- water in the range from 20 to 45% by weight, and humectant in the range from 17 to 50% by weight,
- a component for controlling the rheology in the range from 0,1 to 5% by weight,
- exclusively one or more amphoteric surfactant(s) in an amount in the range from 0,1 to 2,0% by weight,
wherein all of the percents by weight refer to the total mass of the prophylaxis paste.

7. Prophylaxis paste for professional dental use according to one of claims 1-5, wherein the paste comprises
- water in the range from 23 to 35% by weight and humectant in the range from 25 to 42% by weight,
- a cleaning body in the range from 30 to 36% by weight,
- a component for controlling the rheology in the range from 0,1 to 4% by weight,
- exclusively one or more amphoteric surfactant(s) in an amount in the range from 0,80 to 1,20% by weight,
wherein all of the percents by weight refer to the total mass of the prophylaxis paste.

8. Prophylaxis paste for professional dental use according to one of claims 1-7, **characterized in that** it is storage-stable, measured according to the method for determining the centrifugal stability, wherein the determination of the centrifugal stability takes place using a laboratory standard centrifuge (EBA 20® from Hettich) and wherein 3 g of the respective paste are weighed into a centrifuge glass that has a diameter of 15 mm and a length of 100 mm and then centrifuged at 5000 rpm for 15 minutes, wherein the temperature during the centrifugal run is 23°C and wherein, following this method, the investigated pastes are examined visually as to phase homogeneity, and is thixotropically stable, measured according to the method for determining the spreadability, wherein the determination of the spreadability takes place in a climatically controlled room at a temperature of 23°C and a relative atmospheric humidity of 50% by means of a plastic glass plate arrangement, wherein the dimensions of the plates are 60 x 60 x 5 mm and wherein the plate weight is 25 g and wherein a syringe is used to place 0,5 ml of the respective paste onto the first plastic glass plate in the middle of the plate and wherein then the second plastic glass plate is laid on parallel to the first and weighed down in the middle using a cylindrical iron punch weighing 100 g and having a diameter of 33 mm and a height of 13 mm for 10 minutes, and wherein the value of the spreadability is determined as the average of two diameters of the resulting sample cylinder after the weighing down.

9. Kit, comprising tubes, pots or cans comprising one or more soft prophylaxis paste(s) according to one of the preceding claims, **characterized in that** the ratio of the cleaning bodies of the compound based on calcium phosphate to pumice is in a range from 40:1 to 10:1 and the RDA value of the soft paste(s), measured in accordance with DIN EN ISO 11609, is in the range from 5 to 50, preferably in the range from 8 to 30, and/or one or more medium-hard prophylaxis paste(s) according to one of the preceding claims, **characterized in that** the ratio of the cleaning bodies of the compound based on calcium phosphate to pumice is in a range from 7:1 up to and including 3:1 and the RDA value of the medium-hard paste(s), measured in accordance with DIN EN ISO 11609, is in the range from 70 to 150, preferably in the range from 100 to 145, and/or one or more hard prophylaxis paste(s) according to one of the preceding claims, **characterized in that** the ratio of the cleaning bodies of the compound based on calcium phosphate to pumice is in a range from 3:1 to 1:1 and the RDA value of the hard paste(s), measured in accordance with DIN EN ISO 11609, is in the range from 160 to 250, preferably in the range from 170 to 220, and optionally cleaning and/or polishing cups and/or polishing brushes and/or fluoridation agents and/or bleaches and/or remineralizing agents and/or (fissure) sealers.

10. Process for producing a prophylaxis paste according to one of Claims 1 to 8 having the following steps:
a.) providing the base component consisting of water and humectant,
b.) successively dissolving all of the other constituents of the prophylaxis paste that are soluble in this component,
c.) introducing the constituents that are insoluble in this mixture,
d.) processing the mixture to give a homogeneous mass,
e.) degassing the prophylaxis paste composition in vacuo and
f.) discharging the prophylaxis paste into tubes, pots or cans.

11. Use of a composition according to Claims 1 to 8 for producing a prophylaxis paste.

## Revendications

1. Pâte prophylactique pour une utilisation dentaire professionnelle, comprenant
a) un composant de base, contenant de l'eau dans la plage de 15 à 55 % en poids, et un humectant dans la plage de 10 à 60 % en poids,
b) un agent nettoyant dans la plage de 0,5 à 60 % en poids,
c) un composant tampon,
d) un composant pour régler la rhéologie, dans la plage de 0,1 à 7 % en poids,
e) un composant additif dans la plage de 0,001 à 5 % en poids, les additifs étant choisis dans une liste comprenant les stabilisants, les colorants, les pigments et les agents de fluoruration,
la pâte prophylactique contenant en tant qu'agent nettoyant b) un composé à base de phosphate de calcium en combinaison avec de la pierre ponce, et en tant que mouillant
f) comprend exclusivement un ou plusieurs tensioactif(s) amphotère(s) dans la plage de 0,05 à 2,6 % en poids,
tous les pourcentages en poids étant rapportés à la masse totale de la pâte prophylactique,
et
le composant c) étant à base d'un hydrogénophosphate, et maintenant entre 7 et 8 le pH de la pâte prophylactique, déterminée dans une suspension à 10 % (p/p) de la pâte dans l'eau contre une électrode de pH.

2. Pâte prophylactique pour une utilisation dentaire professionnelle selon la revendication 1, dans laquelle le composé à base de phosphate de calcium est l'hydroxylapatite.

3. Pâte prophylactique pour une utilisation dentaire professionnelle selon la revendication 1 ou 2, dans laquelle la proportion des granulométries de la pierre ponce inférieures à 45 µm, exprimée par le refus au tamis en %, est comprise entre 50 et 90 %, de préférence entre 70 et 80 %.

4. Pâte prophylactique pour une utilisation dentaire professionnelle selon l'une des revendications précédentes, dans laquelle le rapport en poids entre les deux composants tampons dihydrogénophosphate de sodium et hydrogénophosphate disodique est de 2:1 à 1:2.

5. Pâte prophylactique pour une utilisation dentaire professionnelle selon l'une des revendications précédentes, dans laquelle le composant pour le réglage de la rhéologie d) est une combinaison de silice pyrogène et d'un dérivé de cellulose.

6. Pâte prophylactique pour une utilisation dentaire professionnelle selon l'une des revendications précédentes, la pâte comprenant :
- de l'eau dans la plage de 20 à 45 % en poids, et un humectant dans la plage de 17 à 50 % en poids,
- un composant pour le réglage de la rhéologie, dans la plage de 0,1 à 5 % en poids,
- exclusivement un ou plusieurs tensioactif(s) amphotère(s) dans la plage de 0,1 à 2,0 % en poids,
tous les pourcentages en poids étant rapportés à la masse totale de la pâte prophylactique.

7. Pâte prophylactique pour une utilisation dentaire professionnelle selon l'une des revendications 1 à 5, la pâte comprenant :
- de l'eau dans la plage de 23 à 35 % en poids et un humectant dans la plage de 25 à 42 % en poids,
- un agent nettoyant dans la plage de 30 à 36 % en poids,
- un composant pour le réglage de la rhéologie dans la plage de 0,1 à 4 % en poids,
- exclusivement un ou plusieurs tensioactif(s) amphotère(s) dans la plage de 0,80 à 1,20 % en poids,
tous les pourcentages en poids étant rapportés à la masse totale de la pâte prophylactique.

8. Pâte prophylactique pour une utilisation dentaire professionnelle selon l'une des revendications 1 à 7, **caractérisée en ce qu'**elle est stable au stockage, comme mesuré par la méthode de détermination de la stabilité à la centrifugation, la détermination de la stabilité à la centrifugation étant réalisée avec une centrifugeuse standard de laboratoire (EBA 20® de Hettich), avec prise d'essai de 3 g de chaque pâte dans un tube de centrifugeuse présentant un diamètre de 15 mm et une longueur de 100 mm ; puis est centrifugée à 5000 tr/min pendant 15 minutes, la température étant de 23°C pendant la marche de la centrifugeuse, les pâtes étudiées faisant, après mise en oeuvre de cette méthode, l'objet d'un examen visuel portant sur l'homogénéité des phases ; et est thixotropiquement résistante, la mesure étant effectuée par la méthode de détermination de l'aptitude à l'étalement, la détermination de l'aptitude à l'étalement étant effectuée dans une chambre climatique à une température de 23°C en présence d'une humidité relative de l'air de 50 %, à l'aide d'un montage de plaques en verre plastique, les dimensions des plaques étant de 60 x 60 x 5 mm, et le poids des plaques étant de 25 g, et 0,5 ml de chaque pâte étant, à l'aide d'une seringue, placé sur la première plaque en verre plastique au milieu de la plaque, la deuxième plaque en verre plastique étant ensuite montée parallèlement à la première et étant, en son milieu, sollicitée pendant 10 minutes avec un tampon de fer cylindrique ayant un poids de 100 g, un diamètre de 33 mm et une hauteur de 13 mm, la valeur de l'aptitude à l'étalement étant déterminée par la moyenne de deux diamètres de la carotte obtenue.

9. Trousse comprenant des tubes, des cupules ou des boîtes contenant une ou plusieurs pâte(s) prophylactique(s) molles selon l'une des revendications précédentes, **caractérisée en ce que** le rapport des agents nettoyants du composé à base de phosphate de calcium à la pierre ponce est compris dans une plage de 40:1 à 10:1, et la valeur RDA mesurée selon DIN EN ISO 11609 de la ou des pâte(s) molle(s) est comprise dans la plage de 5 à 50, de préférence dans la plage de 8 à 30, et une ou plusieurs pâte(s) prophylactique(s) de dureté moyenne selon l'une des revendications précédentes, **caractérisée en ce que** le rapport des agents nettoyants du composé à base de phosphate de calcium à la pierre ponce est compris dans une plage de 7:1 à 3:1 compris, et que la valeur RDA mesurée selon DIN EN ISO 11609 de la ou des pâte(s) de dureté moyenne est comprise dans la plage de 70 à 150, de préférence dans la plage de 100 à 145, et/ou une ou plusieurs pâte(s) prophylactique(s) dure(s) selon l'une des revendications précédentes, **caractérisée en ce que** le rapport des agents nettoyants du composé à base de phosphate de calcium à la pierre ponce est compris dans une plage de 3:1 à 1:1, et la valeur RDA, mesurée selon DIN EN ISO 11609, de la ou des pâte(s) dure(s) est comprise dans la plage de 160 à 250, de préférence dans la plage de 170 à 220, ainsi qu'éventuellement des cupules de nettoyage et/ou de polissage et/ou des brossettes de polissage et/ou des agents de fluoruration et/ou des agents de blanchiment et/ou des agents de reminéralisation et/ou des agents de scellement (de fissures).

10. Procédé de fabrication d'une pâte prophylactique selon l'une des revendications 1 à 8, comportant les étapes suivantes :
a) fourniture d'un composant de base, constitué d'eau et d'un humectant,
b) successivement dissolution de tous les autres constituants de la pâte prophylactique, solubles dans ce composant,
c) introduction des constituants insolubles dans ce mélange,
d) mise en oeuvre du mélange pour donner une masse homogène,
e) désaération sous vide de la composition de pâte prophylactique, et
f) transfert de la pâte prophylactique dans des tubes, des cupules ou des boîtes.

11. Utilisation d'une composition selon l'une des revendications 1 à 8 pour fabriquer une pâte prophylactique.
